# EUROPEAN PATENT APPLICATION

(11) **EP 2 216 068 A1**
(43) Date of publication of application: **11.08.2010**
(21) Application number: 08853533.1
(22) Date of filing: 27.10.2008
(51) Int. Cl.: A61M 25/02, A61M 25/01

(54) **DRIVE DEVICE, MEDICAL DEVICE PROVIDED WITH DRIVE DEVICE, AND TRAINING DEVICE**

(30) Priority: 27.11.2007 JP 2007305670
(71) Applicant: NTN Corporation, Osaka-shi, Osaka 550-0003 (JP)
(72) Inventor: NAGANO, Yoshitaka, Iwata-shi Shizuoka 438-8510 (JP); NISHIO, Yukihiro, Iwata-shi Shizuoka 438-8510 (JP); OZAKI, Takayoshi, Iwata-shi Shizuoka 438-8510 (JP)
(74) Representative: Bockhorni & Kollegen
(86) International application number: PCT/JP2008/069452
(87) International publication number: WO 2009/069413

(57) **Abstract**

A drive device for driving a linear body (1) having flexibility includes a first roller (3), a motor (4) for feeding the linear body (1) in a direction of extension of the linear body (1) by directly transmitting rotation force to the first roller (3) to rotate the first roller (3), a motor drive portion (7) for driving the motor (4) by supplying a current to the motor (4), and a control unit (8) for calculating force applied to the linear body (1) in the direction of extension based on the current supplied to the motor (4).

## Description

### Drive Device, and Medical Apparatus and Training Apparatus Including the Same

### TECHNICAL FIELD

The present invention relates to a drive device as well as a medical apparatus and a training apparatus including the same, and particularly to a drive device for driving a linear body having flexibility as well as a medical apparatus and a training apparatus including the same.

### BACKGROUND ART

A linear body having flexibility has been adopted as a medical appliance to be inserted in a body. For example, a guide wire for guiding a catheter, which is precedently inserted in a vessel such as a blood vessel, a ureter and the like in order to insert the catheter in the vessel such as a blood vessel, a ureter and the like, has been known.

In addition, for example, in treatment for embolizing a cerebral aneurysm from the inside of a cerebral vessel, not only a guide wire but also a delivery wire having a diameter equivalent to that of the guide wire, for leaving in an aneurysm a platinum coil for embolization, is used.

In operating such a medical appliance to be inserted in a body, a medical wire such as a guide wire and a delivery wire is inserted in a vessel in a human body and operated from outside the human body so that the wire is guided to a destination. A vessel in a body is not linear but flexed or branched, and hence skills are required for external guiding operations. In particular, if excessive load is applied to a vessel in a human body during operation, the vessel in the human body may be damaged.

In order to solve such a problem, for example, Japanese Patent Laying-Open No. 10-263089 (Patent Document 1) discloses the following catheter. Specifically, such a catheter that a contact pressure at a sensor portion provided at a tip end of a catheter tube is sensed by a pressure sensing sensor provided in the sensor portion and whether or not an obstacle is present in a direction of travel is sensed based on a sensor output signal from the pressure sensing sensor includes signal auralizing means for converting and auralizing variation in the sensor output signal to voice and sound.
Patent Document 1: Japanese Patent Laying-Open No. 10-263089

### DISCLOSURE OF THE INVENTION

### PROBLEMS TO BE SOLVED BY THE INVENTION

Such a structure that a sensor is attached for use at the tip end of a tube as in the catheter described in Patent Document 1, however, is difficult to realize, in particular in the case of an extremely thin medical wire. In the case of a thin medical wire, in particular, a guide wire and a delivery wire to be inserted in a cerebral vessel in treatment for embolizing a cerebral aneurysm from the inside of a cerebral vessel, the guide wire and the delivery wire each have a diameter of approximately 0.35 mm, and hence it is difficult to attach a small-sized pressure sensor at a tip end portion. In addition, in the case of the delivery wire, since a platinum coil to be detached at the time of embolization is provided at the tip end, it is extremely difficult to attach a small-sized pressure sensor at the tip end portion of the delivery wire. Moreover, it is further difficult to insert wiring in the guide wire and the delivery wire in order to extract a signal from the pressure sensor to the outside of the human body.

Furthermore, since types of medical wires to be used are different depending on surgical operations, it is not cost effective to prepare medical wires with sensor adapted to various surgical operations and cost is increased.

In addition, an output from the pressure sensor provided at the tip end of the medical wire is not necessarily in agreement with kinesthetic sense of an operator at the time of insertion. This is because the vessel in the human body is flexed and insertion resistance of the medical wire is affected by friction or the like with the vessel of the human body. Therefore, the operator should perform an operation for inserting the medical wire based on visual information from a perspective image of the human body and kinesthetic sense information of the insertion resistance of the medical wire held outside the human body with fingers, and the operation becomes complicated.

Therefore, an object of the present invention is to provide a drive device capable of realizing a medical appliance and the like with a simplified structure and preventing an operation from becoming complicated as well as a medical apparatus and a training apparatus including the same.

### MEANS FOR SOLVING THE PROBLEMS

A drive device according to one aspect of the present invention is a drive device for driving a linear body having flexibility, and the drive device includes a first roller, a motor for feeding the linear body in a direction of extension of the linear body by directly transmitting rotation force to the first roller to rotate the first roller, a motor drive portion for driving the motor by supplying a current to the motor, and a control unit for calculating force applied to the linear body in the direction of extension based on the current supplied to the motor.

Preferably, the first roller has a groove formed to extend around an outer circumferential surface of the first roller along a direction of rotation of the first roller.

Preferably, the drive device further includes a second roller, and the linear body is sandwiched between the first roller and the second roller and fed in the direction of extension as the first roller and the second roller rotate.

Further preferably, the drive device further includes an elastic body having an inner circumferential surface in contact with an outer circumferential surface of the first roller and formed to cover the outer circumferential surface of the first roller, and the linear body is sandwiched between the elastic body and the second roller.

Further preferably, the drive device further includes a first elastic body having an inner circumferential surface in contact with an outer circumferential surface of the first roller and formed to cover the outer circumferential surface of the first roller, and a second elastic body having an inner circumferential surface in contact with an outer circumferential surface of the first elastic body and formed to cover the outer circumferential surface of the first elastic body, the linear body is sandwiched between the second elastic body and the second roller, and the second elastic body is higher in hardness than the first elastic body.

Further preferably, the drive device further includes a pressure adjustment portion for adjusting force with which the first roller and the second roller sandwich the linear body.

A medical apparatus according to one aspect of the present invention includes a linear body having flexibility and a drive device for driving the linear body, and the drive device includes a first roller, a motor for feeding the linear body in a direction of extension of the linear body by directly transmitting rotation force to the first roller to rotate the first roller, a motor drive portion for driving the motor by supplying a current to the motor, and a control unit for calculating force applied to the linear body in the direction of extension based on the current supplied to the motor.

Preferably, the linear body is provided with an embolization coil for embolizing a cerebral aneurysm at its tip end.

Preferably, the linear body is a guide wire for guiding a catheter to a destination.

A training apparatus according to one aspect of the present invention includes a linear body having flexibility and a drive device for driving the linear body, and the drive device includes a first roller, a motor for feeding the linear body in a direction of extension of the linear body by directly transmitting rotation force to the first roller to rotate the first roller, a motor drive portion for driving the motor by supplying a current to the motor, and a control unit for calculating force applied to the linear body in the direction of extension based on the current supplied to the motor.

### EFFECTS OF THE INVENTION

According to the present invention, a medical appliance and the like can be realized with a simplified structure and an operation can be prevented from becoming complicated.

### BRIEF DESCRIPTION OF THE DRAWINGS

Fig. 1 is a diagram showing a structure of a drive device according to Embodiment 1 of the present invention.
Fig. 2 is a perspective view showing a linear body, a pressing roller, a drive roller, and a motor in the drive device according to Embodiment 1 of the present invention.
Fig. 3 is a diagram showing one example of a method of controlling a motor in the drive device according to Embodiment 1 of the present invention.
Fig. 4 is a diagram showing another example of a method of controlling a motor in the drive device according to Embodiment 1 of the present invention.
Fig. 5 is a diagram showing a structure of a drive device according to Embodiment 2 of the present invention.
Fig. 6 is a perspective view showing a linear body, a pressing roller, a drive roller, and a motor in the drive device according to Embodiment 2 of the present invention.
Fig. 7 is a diagram showing a structure of a drive device according to Embodiment 3 of the present invention.
Fig. 8 is a diagram showing a structure of a drive device according to Embodiment 4 of the present invention.
Fig. 9 is a diagram showing a structure of a drive device according to Embodiment 5 of the present invention.
Fig. 10 is a diagram showing a structure of a medical apparatus according to Embodiment 6 of the present invention.
Fig. 11 is a diagram showing an example where a linear body is a guide wire in the medical apparatus according to Embodiment 6 of the present invention.
Fig. 12 is a diagram showing an example where a linear body is a delivery wire in the medical apparatus according to Embodiment 6 of the present invention.
Fig. 13 is a diagram showing a structure of a training apparatus according to Embodiment 7 of the present invention.

### DESCRIPTION OF THE REFERENCE SIGNS

1 linear body; 2 pressing roller; 3, 73 drive roller; 4 motor; 5 groove; 6 rolling bearing; 7 motor drive portion; 8 control unit; 9 rolling bearing; 11 to 13 elastic body; 21 cam motor; 22 cam; 23 spring; 24 fixed portion; 31 catheter; 32 Y-connector; 33, 43 cable; 35 display; 36 speaker; 37 human body; 38 cerebral vessel; 39 cerebral aneurysm; 41 simulator; 51 current conversion circuit; 52 current control circuit; 53 current detection circuit; 54 embolization coil; 61 encoder; 62 speed detection circuit; 63 speed control circuit; 71 pressure adjustment portion; 101 to 105 drive device; 201 medical apparatus; 301 training apparatus; SH1, SH2 rotation shaft; D1, D2 deformed portion; P1, P2 input port; and P3 output port.

### BEST MODES FOR CARRYING OUT THE INVENTION

An embodiment of the present invention will be described hereinafter with reference to the drawings. In the drawings, the same or corresponding elements have the same reference characters allotted, and description thereof will not be repeated.

### <Embodiment 1>

### [Structure and Basic Operation]

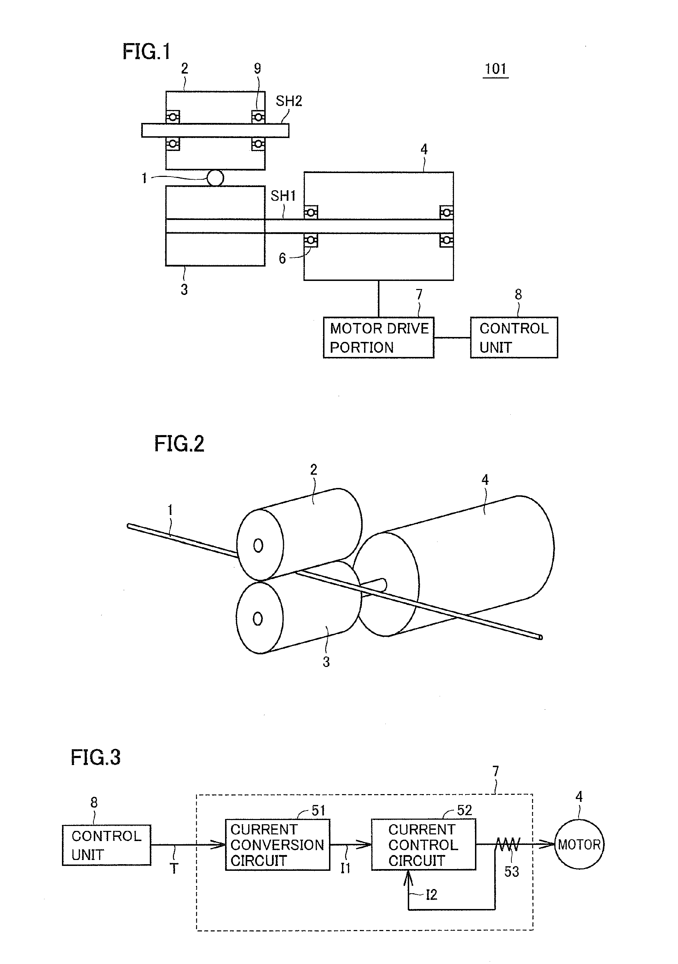

Fig. 1 is a diagram showing a structure of a drive device according to Embodiment 1 of the present invention.

Referring to Fig. 1, a drive device 101 includes a pressing roller 2, a drive roller 3, a motor 4, a rolling bearing 6, a motor drive portion 7, and a control unit 8. Motor 4 includes a rotation shaft SH1 and rolling bearing 6. Pressing roller 2 includes a rotation shaft SH2 and a rolling bearing 9.

Control unit 8 controls rotation of motor 4 by providing a rotation command to motor drive portion 7.

Motor drive portion 7 drives motor 4 by supplying a motor current to motor 4 based on the rotation command from control unit 8.

Motor 4 rotates based on the motor current supplied from motor drive portion 7 and directly transmits that rotation force to drive roller 3 without a speed reduction mechanism being interposed.

Fig. 2 is a perspective view showing a linear body, the pressing roller, the drive roller, and the motor in the drive device according to Embodiment 1 of the present invention.

Referring to Fig. 2, drive roller 3 rotates with rotation force transmitted from motor 4. Pressing roller 2 is arranged opposite to drive roller 3, with respect to a linear body 1. Linear body 1 is sandwiched between pressing roller 2 and drive roller 3.

In addition, drive roller 3 transmits rotation force to linear body 1 as a result of friction force generated between pressing roller 2, drive roller 3 and linear body 1. Linear body 1 is thus fed in a direction of extension of linear body 1 as pressing roller 2 and drive roller 3 rotate.

Referring again to Fig. 1, control unit 8 calculates drive force applied to linear body 1, that is, force applied to linear body 1 in the direction of extension, based on a motor current supplied from motor drive portion 7 to motor 4. More specifically, control unit 8 estimates force applied to linear body 1 in the direction of extension by multiplying a motor current by a torque coefficient specific to motor 4 and by dividing a result of multiplication by a radius of drive roller 3.

In the drive device according to Embodiment 1 of the present invention, since motor 4 does not include a speed reduction mechanism, motor 4 rotates slowly. Thus, cogging torque of motor 4 is desirably as close as possible to zero. Therefore, motor 4 is desirably a coreless motor. In addition, motor 4 is desirably an AC servo motor or a multipolar DC motor less in torque fluctuation involved with rotation, so that motor torque, that is, rotation force, is in proportion to a motor current even though a rotation speed is low.

Friction in a rotation force transmission path from rotation shaft SH1 of motor 4 to linear body 1 causes loss of drive force of motor 4. Namely, since friction in this transmission path turns out to be hysteresis for actual drive force at the time when drive force is estimated based on a motor current, friction in rotation of motor 4 is desirably zero.

In the drive device according to Embodiment 1 of the present invention, motor 4 includes rolling bearing 6 supporting rotation shaft SH1. According to such a structure, friction in the rotation force transmission path from rotation shaft SH1 of motor 4 to linear body 1 can be reduced.

Pressing roller 2 includes rolling bearing 9 supporting rotation shaft SH2. According to such a structure, friction in the rotation force transmission path from rotation shaft SH2 of pressing roller 2 to linear body 1 can be reduced.

Fig. 3 is a diagram showing one example of a method of controlling a motor in the drive device according to Embodiment 1 of the present invention.

Referring to Fig. 3, motor drive portion 7 includes a current conversion circuit 51, a current control circuit 52, and a current detection circuit 53.

Control unit 8 controls rotation of motor 4 by providing a rotation command indicating drive force T to motor drive portion 7.

Current conversion circuit 51 converts drive force T indicated by a rotation command from motor drive portion 7 into a current value I1 and outputs the value to current control circuit 52.

Current detection circuit 53 detects a value I2 of motor current supplied to motor 4 and outputs the value to current control circuit 52.

Current control circuit 52 controls a motor current to be supplied to motor 4 such that current value I2 received from current detection circuit 53 is equal to current value I1 received from current conversion circuit 51.

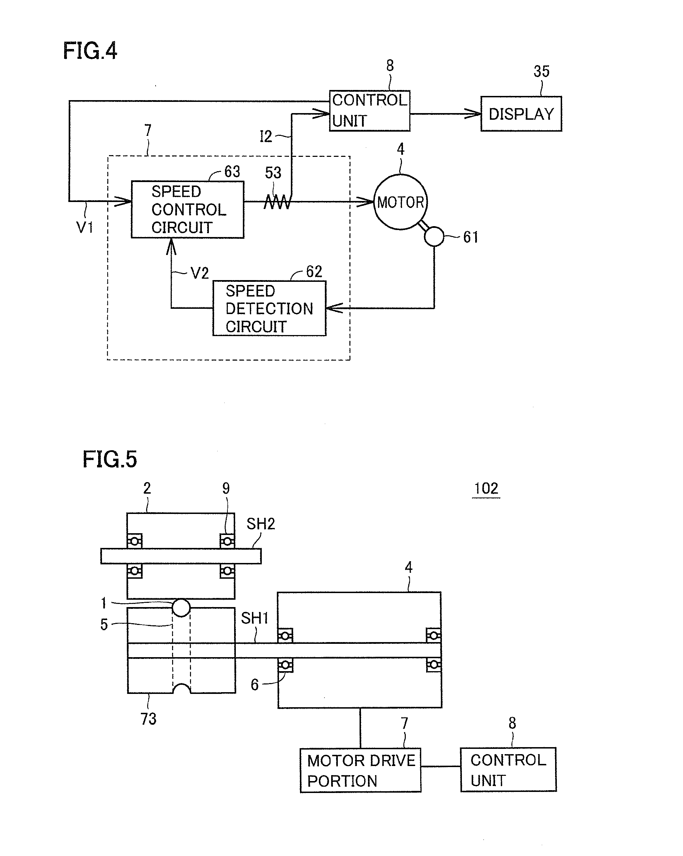

Fig. 4 is a diagram showing another example of a method of controlling a motor in the drive device according to Embodiment 1 of the present invention.

Referring to Fig. 4, drive device 101 further includes an encoder 61 and a display 35. Motor drive portion 7 includes a speed detection circuit 62, a speed control circuit 63, and current detection circuit 53.

Control unit 8 controls rotation of motor 4 by providing a rotation command indicating a rotation speed V1 to motor drive portion 7.

Encoder 61 detects an angle of rotation or the like of rotation shaft SH1 of motor 4 and outputs the angle or the like as rotation position information to speed detection circuit 62.

Speed detection circuit 62 detects a rotation speed V2 of motor 4 based on the rotation position information received from encoder 61 and outputs the speed to speed control circuit 63.

Speed control circuit 63 controls a motor current to be supplied to motor 4 such that rotation speed V2 received from speed detection circuit 62 is equal to rotation speed V1 received from control unit 8.

Current detection circuit 53 detects value I2 of motor current supplied to motor 4 and outputs the value to control unit 8.

Control unit 8 calculates magnitude of force applied to linear body 1 in the direction of extension based on current value I2 received from current detection circuit 53 and notifies display 35 of the magnitude. Display 35 displays the magnitude of force received from control unit 8 on a screen.

The catheter described in Patent Document 1 has been disadvantageous in that a structure and an operation of a medical appliance or the like are complicated.

In a measurement device according to Embodiment 1 of the present invention, however, motor 4 rotates based on a motor current supplied from motor drive portion 7 and rotation force is directly transmitted to drive roller 3 to thereby rotate drive roller 3. Thus, linear body 1 is fed in the direction of extension of linear body 1. Control unit 8 calculates force applied to linear body 1 in the direction of extension based on the motor current. According to such a structure, force in the direction of extension of linear body 1 around a hand of an operator can be detected. In addition, since it is not necessary to attach a pressure sensor at the tip end of a medical wire, it is not necessary to prepare a medical wire with sensor or the like for each type of surgical operation.

Therefore, the drive device according to Embodiment 1 of the present invention can realize a medical appliance or the like with a simplified structure and prevent an operation from becoming complicated.

In addition, as described previously, since output of the pressure sensor attached to the tip end of the medical wire is not necessarily in agreement with kinesthetic sense of the operator at the time of insertion thereof, only the operator who is actually inserting the medical wire can know his/her kinesthetic sense about the medical wire. Therefore, the catheter described in Patent Document 1 has been disadvantageous in that manipulation cannot quantitatively be transferred to a less experienced operator. The measurement device according to Embodiment 1 of the present invention, however, measures force applied to linear body 1 in the direction of extension, so that the operation performed by a skilled operator can be quantified and manipulation of the less experienced operator can quickly be improved.

In addition, in the drive device according to Embodiment 1 of the present invention, as motor 4 directly transmits rotation force to drive roller 3 without a speed reduction mechanism being interposed, there is no friction in rotation of the speed reduction mechanism. Therefore, force applied to linear body 1 in the direction of extension can readily be estimated based on the motor current determining drive force of motor 4.

Since linear body 1 is thin and it is inserted in a human body, force in the direction of extension tolerable for insertion is small. Therefore, motor 4 can sufficiently drive linear body 1 without a speed reduction mechanism.

Another embodiment of the present invention will now be described with reference to the drawings. In the drawings, the same or corresponding elements have the same reference characters allotted, and description thereof will not be repeated.

### <Embodiment 2>

The present embodiment relates to a drive device in which a mechanism for rotating a linear body is modified as compared with the drive device according to Embodiment 1. The drive device is the same as the drive device according to Embodiment 1, except for disclosure below.

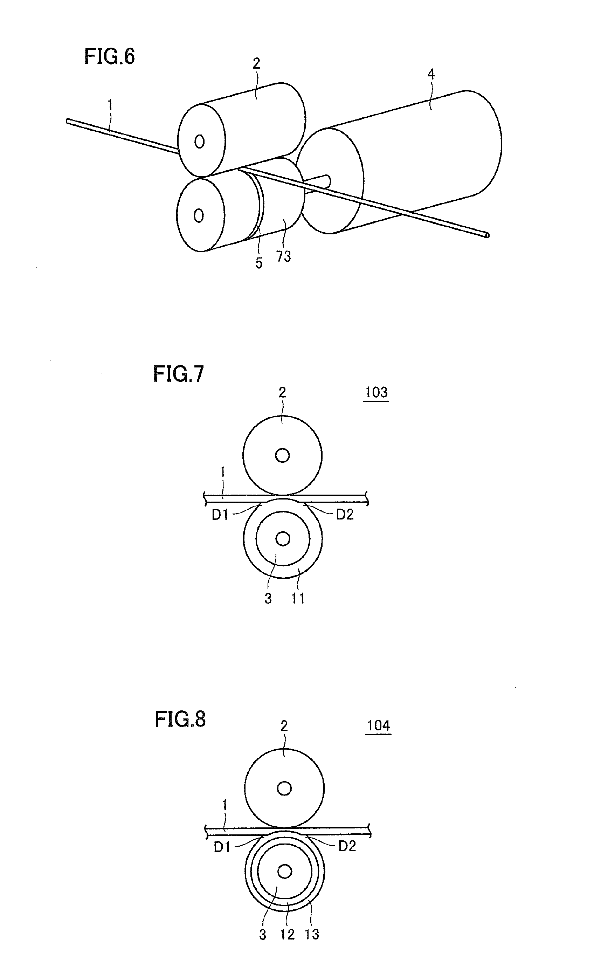

Fig. 5 is a diagram showing a structure of the drive device according to Embodiment 2 of the present invention. Fig. 6 is a perspective view showing the linear body, the pressing roller, a drive roller, and the motor in the drive device according to Embodiment 2 of the present invention.

Referring to Figs. 5 and 6, a drive device 102 includes a drive roller 73 instead of drive roller 3, as compared with the drive device according to Embodiment 1 of the present invention.

Drive roller 73 has a groove 5 provided in an outer circumferential surface of drive roller 73 and formed to extend around the outer circumferential surface of drive roller 73 along a direction of rotation of drive roller 73.

According to such a structure, since a coefficient of friction between drive roller 3 and linear body 1 can be made larger, rotation force from motor 4 can sufficiently be transmitted to linear body 1. Here, as pressing roller 2 does not transmit rotation force from motor 4, the need to provide a groove therein is low. In addition, according to such a structure that a groove is provided only on drive roller 3 side, accurate registration of positions of pressing roller 2 and drive roller 3 in a direction of rotation shaft is not necessary.

As a structure and an operation are otherwise the same as those in the drive device according to Embodiment 1, detailed description will not be repeated here. Therefore, the drive device according to Embodiment 2 of the present invention can realize a medical appliance or the like with a simplified structure and prevent an operation from becoming complicated.

Another embodiment of the present invention will now be described with reference to the drawings. In the drawings, the same or corresponding elements have the same reference characters allotted, and description thereof will not be repeated.

### <Embodiment 3>

The present embodiment relates to a drive device in which arrangement for increasing a coefficient of friction between the drive roller and the linear body is added as compared with the drive device according to Embodiment 1. The drive device is the same as the drive device according to Embodiment 1, except for disclosure below.

Fig. 7 is a diagram showing a structure of the drive device according to Embodiment 3 of the present invention.

Referring to Fig. 7, a drive device 103 further includes an elastic body 11, as compared with the drive device according to Embodiment 1 of the present invention.

Elastic body 11 is made, for example, of urethane rubber. Elastic body 11 has an inner circumferential surface in contact with the outer circumferential surface of drive roller 3 and it is formed to cover the outer circumferential surface of drive roller 3.

Linear body 1 is sandwiched between pressing roller 2 and elastic body 11. Deformed portions D1 and D2 are formed in elastic body 11 due to force generated between elastic body 11 and linear body 1.

In addition, drive roller 3 transmits rotation force as a result of friction force generated between pressing roller 2, elastic body 11 and linear body 1. Linear body 1 is thus fed in the direction of extension as pressing roller 2 and drive roller 3 rotate.

In the drive device according to Embodiment 3 of the present invention, with the structure including elastic body 11, a coefficient of friction between drive roller 3 and linear body 1 can be made larger. Therefore, force with which linear body 1 is sandwiched between pressing roller 2 and drive roller 3, that is, force in a direction of width of linear body 1 applied from pressing roller 2 to linear body 1, can be reduced.

Here, as force applied by pressing roller 2 to linear body 1 in the direction of width is greater, friction in rotation of rolling bearing 9 in pressing roller 2 increases. Therefore, force applied by pressing roller 2 to linear body 1 in the direction of width is preferably small. In addition, when elastic body 11 is deformed by the force applied by pressing roller 2, rotation resistance of drive roller 3 increases in accordance with magnitude of that force. Then, in estimating drive force for linear body 1 based on a motor current, hysteresis for actual drive force is generated and hence error in estimation of drive force for linear body 1 is caused. Thus, urethane rubber forming elastic body 11 desirably has high hardness. Urethane rubber has hardness in a range from 10 to 99 and desirably not lower than 90.

As a structure and an operation are otherwise the same as those in the drive device according to Embodiment 1, detailed description will not be repeated here. Therefore, the drive device according to Embodiment 3 of the present invention can realize a medical appliance or the like with a simplified structure and prevent an operation from becoming complicated.

Another embodiment of the present invention will now be described with reference to the drawings. In the drawings, the same or corresponding elements have the same reference characters allotted, and description thereof will not be repeated.

### <Embodiment 4>

The present embodiment relates to a drive device in which a structure of an elastic body is improved as compared with the drive device according to Embodiment 3. The drive device is the same as the drive device according to Embodiment 1, except for disclosure below.

Fig. 8 is a diagram showing a structure of the drive device according to Embodiment 4 of the present invention.

Referring to Fig. 8, a drive device 104 further includes elastic bodies 12 and 13, as compared with the drive device according to Embodiment 1 of the present invention.

Elastic bodies 12 and 13 are made, for example, of urethane rubber. Elastic body 12 has an inner circumferential surface in contact with the outer circumferential surface of drive roller 3 and it is formed to cover the outer circumferential surface of drive roller 3. Elastic body 13 has an inner circumferential surface in contact with an outer circumferential surface of elastic body 12 and it is formed to cover the outer circumferential surface of elastic body 12. Elastic body 13 is higher in hardness than elastic body 12.

Linear body 1 is sandwiched between pressing roller 2 and elastic body 13. Deformed portions D 1 and D2 are formed in elastic body 13 due to force generated between elastic body 13 and linear body 1.

In addition, drive roller 3 transmits rotation force as a result of friction force generated between pressing roller 2, elastic body 13 and linear body 1. Linear body 1 is thus fed in the direction of extension as pressing roller 2 and drive roller 3 rotate.

In the drive device according to Embodiment 4 of the present invention, as drive roller 3 rotates, deformed portions D1 and D2 in elastic body 13 move to elastic body 12 softer than elastic body 13. Thus, since friction in rotation, that is, rotation resistance, of drive roller 3 caused by deformed portions D1 and D2 decreases, accuracy in estimation of drive force for linear body 1 based on a motor current can be improved.

As a structure and an operation are otherwise the same as those in the drive device according to Embodiment 1, detailed description will not be repeated here. Therefore, the drive device according to Embodiment 4 of the present invention can realize a medical appliance or the like with a simplified structure and prevent an operation from becoming complicated.

Another embodiment of the present invention will now be described with reference to the drawings. In the drawings, the same or corresponding elements have the same reference characters allotted, and description thereof will not be repeated.

### <Embodiment 5>

The present embodiment relates to a drive device in which arrangement for adjusting force to be applied to linear body 1 in a direction of width by pressing roller 2 is added as compared with the drive device according to Embodiment 3. The drive device is the same as the drive device according to Embodiment 3, except for disclosure below.

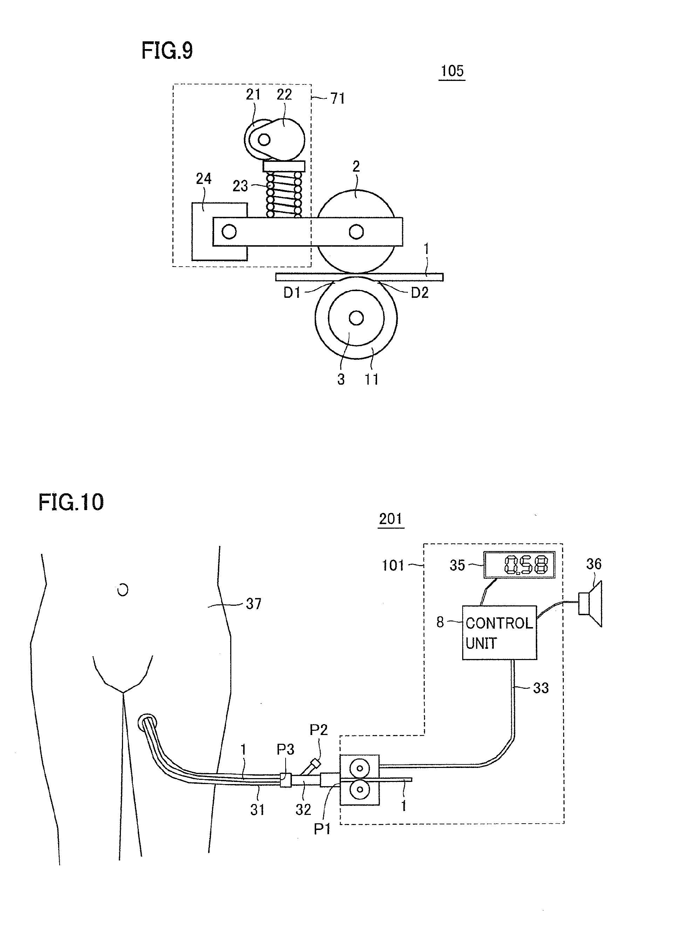

Fig. 9 is a diagram showing a structure of the drive device according to Embodiment 5 of the present invention.

Referring to Fig. 9, a drive device 105 further includes a pressure adjustment portion 71, as compared with the drive device according to Embodiment 3 of the present invention. Pressure adjustment portion 71 includes a cam motor 21, a cam 22, a spring 23, and a fixed portion 24.

Pressure adjustment portion 71 adjusts force with which pressing roller 2 and elastic body 11 sandwiches linear body 1, by adjusting force applied by pressing roller 2 to linear body 1 in the direction of width.

Cam motor 21 increases and decreases elastic force of spring 23, by rotating cam 22. Spring 23 is provided on fixed portion 24. Fixed portion 24 transmits elastic force of spring 23 to pressing roller 2.

When large drive force for linear body 1 in the direction of extension is required, control unit 8 increases elastic force of spring 23 by rotating cam motor 21 clockwise. Thus, force applied by pressing roller 2 to linear body 1 in the direction of width is increased.

On the other hand, when large drive force for linear body 1 in the direction of extension is not required, control unit 8 controls cam motor 21 to decrease elastic force of spring 23, to thereby decrease force applied by roller 2 to linear body 1 in the direction of width.

According to the structure above, when force applied to linear body 1 in the direction of extension is small, friction in rotation of a rotation bearing included in pressing roller 2, motor 4 and the like can be made smaller and hence force applied to linear body 1 in the direction of extension can accurately be estimated based on the motor current.

As a structure and an operation are otherwise the same as those in the drive device according to Embodiment 3, detailed description will not be repeated here. Therefore, the drive device according to Embodiment 5 of the present invention can realize a medical appliance or the like with a simplified structure and prevent an operation from becoming complicated.

Another embodiment of the present invention will now be described with reference to the drawings. In the drawings, the same or corresponding elements have the same reference characters allotted, and description thereof will not be repeated.

### <Embodiment 6>

An example where the drive device according to the present invention is used for actual medical practice such as treatment or examination will now be described.

Fig. 10 is a diagram showing a structure of a medical apparatus according to Embodiment 6 of the present invention.

Referring to Fig. 10, a medical apparatus 201 includes drive device 101, linear body 1, a catheter 31, a Y-connector 32, and a speaker 36. Y-connector 32 includes input ports P1 and P2 and an output port P3. Drive device 101 is connected to input port P1 of Y-connector 32.

Catheter 31 is connected to output port P3 of Y-connector 32. Linear body 1 is inserted in catheter 31. Linear body 1 is guided to a destination in a human body 37 by means of drive device 101.

By measuring force applied to linear body 1 in the direction of extension, reaction force of the measured force, that is, load applied to the vessel in human body 37 by linear body 1, can be measured. Namely, contact of the tip end of the medical appliance with an inner wall of a vessel can be sensed, and hence application of excessive load onto the vessel in the body can be prevented.

In addition, a medicine can be injected through input port P2 of Y-connector 32. For example, physiological saline for reducing friction between catheter 31 and linear body 1 can be injected through input port P2. In addition, after catheter 31 inserted in the blood vessel is guided to the destination in human body 37, a contrast medium can be injected through input port P2 so that the contrast medium can be injected to the destination in human body 37.

Control unit 8 includes a not-shown operation portion for recognizing a user's operation and provides a command indicating a rotation speed or the like of motor 4 to motor drive portion 7 based on the user's operation.

Control unit 8 and motor drive portion 7 are connected to each other through a cable 33. Control unit 8 causes display 35 to display magnitude of force applied to linear body 1 in the direction of extension on a screen thereof.

In addition, control unit 8 may be configured to notify an operator of drive force for linear body 1 not only through the sense of sight but also through the sense of hearing such as tone and volume. For example, control unit 8 controls speaker 36 to issue alarm sound when force applied to linear body 1 in the direction of extension exceeds a prescribed value.

According to such a configuration, an operator who is a doctor can perform medical practice while checking force for insertion of the linear body, so that medical malpractice due to mishandling of the linear body can be prevented. In addition, a guide wire, a delivery wire and the like that have conventionally been used for medical practice can be used as they are.

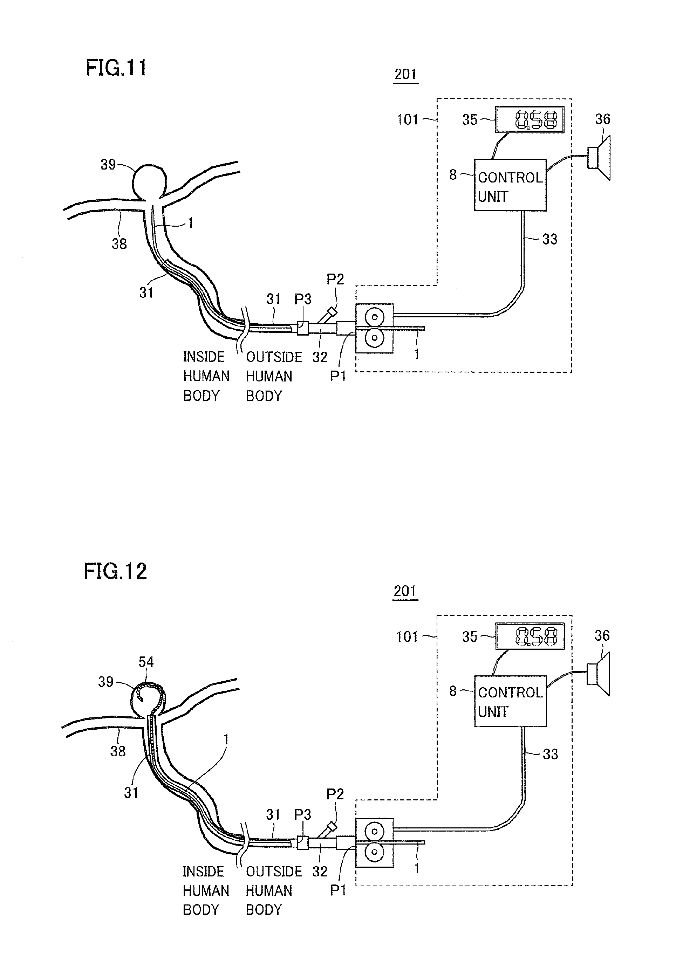

Fig. 11 is a diagram showing an example where the linear body is a guide wire in the medical apparatus according to Embodiment 6 of the present invention.
Fig. 12 is a diagram showing an example where the linear body is a delivery wire in the medical apparatus according to Embodiment 6 of the present invention.

The guide wire and the delivery wire are used, for example, as follows. Namely, referring to Fig. 11, after guide wire 1 is used to guide catheter 31 to a destination, that is, a cerebral aneurysm 39 of a cerebral vessel 38, guide wire 1 alone is pulled out.

Referring to Fig. 12, thereafter, delivery wire 1 with an embolization coil 54 being attached to the front end is inserted in catheter 31. When embolization coil 54 was successfully rested at the destination, embolization coil 54 is detached from delivery wire 1 and delivery wire 1 alone is pulled out of catheter 31.

Another embodiment of the present invention will now be described with reference to the drawings. In the drawings, the same or corresponding elements have the same reference characters allotted, and description thereof will not be repeated.

### <Embodiment 7>

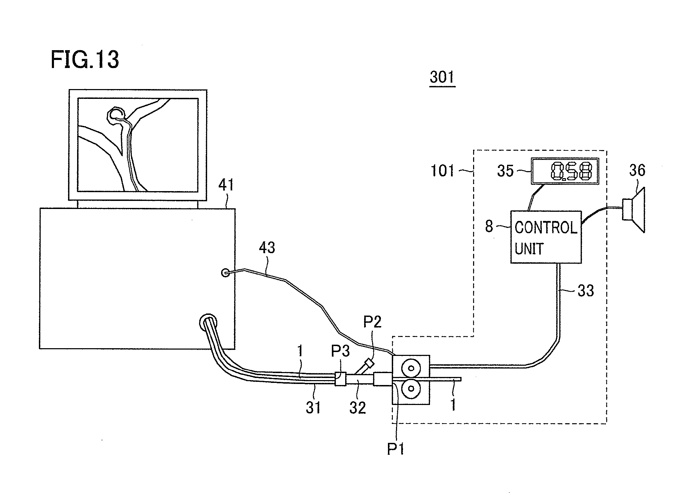

Fig. 13 is a diagram showing a structure of a training apparatus according to Embodiment 7 of the present invention.

Referring to Fig. 13, a training apparatus 301 includes drive device 101, linear body 1, catheter 31, Y-connector 32, speaker 36, a simulator 41, and a cable 43.

Simulator 41 simulates a human body and displays an image equivalent to a perspective image of a vessel in a human body. An operator in training who uses training apparatus 301 operates linear body 1 through a not-shown operation portion in control unit 8 while viewing an image displayed on simulator 41. Simulator 41 varies the insertion resistance of inserted linear body 1. Magnitude of resistance during operation, i.e., force applied to linear body 1 that is measured by drive device 101 in the direction of extension, is displayed on display 35 and also transmitted to simulator 41 through cable 43. Simulator 41 varies the insertion resistance of linear body 1 based on magnitude of the transmitted force.

Though drive device 101 is separate from simulator 41 in Fig. 13, drive device 101 and simulator 41 may integrally be structured. In addition, a structure may be such that magnitude of force applied to linear body 1 in the direction of extension is additionally displayed in a simulated perspective image displayed on simulator 41, instead of display 35.

According to such a structure, operations performed by a skilled operator can be quantified and manipulation of a less experienced operator can quickly be improved.
In addition, operations performed by the operator can be recorded as records of a surgical operation, together with a perspective image.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

## Claims

1. A drive device for driving a linear body (1) having flexibility, comprising:
a first roller (3);
a motor (4) for feeding said linear body (1) in a direction of extension of said linear body (1) by directly transmitting rotation force to said first roller (3) to rotate said first roller (3);
a motor drive portion (7) for driving said motor (4) by supplying a current to said motor (4); and
a control unit (8) for calculating force applied to said linear body (1) in the direction of extension based on the current supplied to said motor (4).

2. The drive device according to claim 1, wherein
said first roller (3) has a groove (5) formed to extend around an outer circumferential surface of said first roller (3) along a direction of rotation of said first roller (3).

3. The drive device according to claim 1, further comprising a second roller (2), wherein
said linear body (1) is sandwiched between said first roller (3) and said second roller (2) and fed in the direction of extension as said first roller (3) and said second roller (2) rotate.

4. The drive device according to claim 3, further comprising an elastic body (11) having an inner circumferential surface in contact with an outer circumferential surface of said first roller (3) and formed to cover the outer circumferential surface of said first roller (3), wherein
said linear body (1) is sandwiched between said elastic body (11) and said second roller (2).

5. The drive device according to claim 3, further comprising:
a first elastic body (12) having an inner circumferential surface in contact with an outer circumferential surface of said first roller (3) and formed to cover the outer circumferential surface of said first roller (3); and
a second elastic body (13) having an inner circumferential surface in contact with an outer circumferential surface of said first elastic body (12) and formed to cover the outer circumferential surface of said first elastic body (12), wherein
said linear body (1) is sandwiched between said second elastic body (13) and said second roller (2), and
said second elastic body (13) is higher in hardness than said first elastic body (12).

6. The drive device according to claim 3, further comprising a pressure adjustment portion (71) for adjusting force with which said first roller (3) and said second roller (2) sandwich said linear body (1).

7. A medical apparatus, comprising:
a linear body (1) having flexibility; and
a drive device (101 to 105) for driving said linear body (1), and
said drive device (101 1 to 105) including
a first roller (3),
a motor (4) for feeding said linear body (1) in a direction of extension of said linear body (1) by directly transmitting rotation force to said first roller (3) to rotate said first roller (3),
a motor drive portion (7) for driving said motor (4) by supplying a current to said motor (4), and
a control unit (8) for calculating force applied to said linear body (1) in the direction of extension based on the current supplied to said motor (4).

8. The medical apparatus according to claim 7, wherein
said linear body (1) is provided with an embolization coil for embolizing a cerebral aneurysm at its tip end.

9. The medical apparatus according to claim 7, wherein
said linear body (1) is a guide wire for guiding a catheter (31) to a destination.

10. A training apparatus, comprising:
a linear body (1) having flexibility; and
a drive device (101 to 105) for driving said linear body (1), and
said drive device including
a first roller (3),
a motor (4) for feeding said linear body (1) in a direction of extension of said linear body (1) by directly transmitting rotation force to said first roller (3) to rotate said first roller (3),
a motor drive portion (7) for driving said motor (4) by supplying a current to said motor (4), and
a control unit (8) for calculating force applied to said linear body (1) in the direction of extension based on the current supplied to said motor (4).
